# FASCICULE DE BREVET EUROPEEN

(11) **EP 1 587 793 B1**
(45) Date de publication et mention de la délivrance du brevet: **28.11.2007**
(21) Numéro de dépôt: 04705107.3
(22) Date de dépôt: 26.01.2004
(51) Int. Cl.: C07D 219/02, C12Q 1/04, C12Q 1/37

(54) **SUBSTRATS ENZYMATIQUES, MILIEUX DE CULTURE LES CONTENANT, UTILISATION POUR DETECTER UNE ACTIVITE AMINOPEPTIDASE ET/OU DIFFERENCIER DES BACTERIES À GRAM POSITIFS DES BACTERIES À GRAM NEGATIFS**
ENZYMSUBSTRATE, DIESE ENTHALTENDE KULTURMEDIEN UND DEREN VERWENDUNG ZUM NACHWEIS VON AMINOPEPTIDASEAKTIVITÄT UND/ODER ZUR DIFFERENZIERUNG VON GRAMPOSITIVEN BAKTERIEN VON GRAMNEGATIVENBAKTERIEN
ENZYME SUBSTRATES, CULTURE MEDIA CONTAINING SAME AND USE THEREOF IN ORDER TO DETECT AMINOPEPTIDASE ACTIVITY AND/OR DIFFERENTIATE GRAM-POSITIVE BACTERIA FROM GRAM-NEGATIVE BACTERIA

(30) Priorité: 29.01.2003 FR 0300953
(43) Date de publication de la demande: 26.10.2005
(73) Titulaire: BIOMERIEUX, 69280 Marcy-L'Etoile (FR)
(72) Inventeur: JAMES, Arthur, Rothbury, Northumberland NE65 7PT (GB); RIGBY, Annette, Haltwhistle, Northumberland NE49 NS (GB)
(74) Mandataire: Bitaud, Valérie Marie-Odile
(86) Numéro de dépôt international: PCT/FR2004/050031
(87) Numéro de publication internationale: WO 2004/069804

(56) Documents cités:
- EP-A- 0 270 946
- WO-A-01/09372
- WO-A-98/04735
- WO-A-99/38995
- FR-A- 2 659 982
- US-A- 4 457 866

## Description

La présente invention concerne de nouveaux substrats enzymatiques chromogènes pour la détection d'activité aminopeptidase. Ces substrats sont utilisables dans les applications comportant une étape d'hydrolyse enzymatique produisant un signal physico-chimique notamment en microbiologie, biochimie, immunologie, biologie moléculaire, histologie, etc. L'invention concerne également des milieux de culture contenant de tels substrats, l'utilisation des substrats ou des milieux pour la détection d'activités aminopeptidases et/ou la différentiation de bactéries à Gram positif par rapport à des bactéries à Gram négatif et des procédés d'utilisation.

Comparativement aux substrats existants, la plupart fluorigènes, ces nouveaux substrats peuvent être utilisés notamment en milieux gélifiés pour la détection de micro-organismes car ils produisent une coloration ne diffusant pas dans le milieu réactionnel donc concentrée au niveau des colonies.

Des substrats chromogéniques enzymatiques pour la détection d'activité aminopeptidase ne diffusant pas sont décrits et déjà connus de l'état de la technique. Ainsi, de tels substrats sont couverts par les demandes de brevet WO-A-98/04735 et WO-A-99/38995 déposées par la Demanderesse. Néanmoins, ces substrats présentent différents inconvénients : leur synthèse est difficile, la pureté est réduite et les rendements faibles. De plus, pour une utilisation en milieux de culture, il faut définir une composition de milieu très précise pour observer une couleur. Aucun des autres substrats actuellement décrits ne peut être utilisé en milieux solides pour la détection de micro-organismes en cultures mixtes.

D'autre part, des molécules à base d'acridine sont connues. Elles sont utilisées pour :
- leurs propriétés de colorant, voir par exemples Rapposch S. et al. J. Dairy Sci. 2000 Dec ; 83 (12) : 2753-2758, ou bien par exemple Giorgio A. et al. Microbiologica 1989 Jan ; 12 (1) : 97-100,
- leurs propriétés chimiothérapeutiques, par exemple Costes N. et al. J. Med. Chem. 2000 Jun 15 ; 43 (12) : 2395-2402, ou
- effectuer des intercalations dans l'ADN, par exemples Okwumabua O. et al. Res. Microbiol. 1992 Feb ; 143 (2) : 183-189 ou encore par exemple Schelhorn T. et al. Cell. Mol. Biol. 1992 Jul; 38 (4) : 345-365.

Le brevet EP-B-0270.946 propose des substrats enzymatiques chromogéniques à base d'acridinone, qui est un dérivé de l'acridine. Le radical, qui peut être clivé par une enzyme, est présent au niveau de la position 7 du groupement acridine. Sa structure ne permet la révélation que des activités enzymatiques suivantes: estérases et glycosidases.

Le brevet US 4,457,866 décrit des peptides chromogéniques et leur utilisation comme substrats enzymatiques pour la détection d'enzymes protéolytiques. La spécificité de ces substrats peut être modulée en fonction des acides aminés introduits dans les peptides.

Le document WO 01/09372 propose des composés chemi-luminescents à base d'acridinine ou de ses dérivés, pouvant être utilisés comme substrats d'enzymes hydrolytiques.

Conformément à la présente invention, il est proposé de nouveaux substrats chromogéniques enzymatiques pour la détection chez des micro-organismes d'une activité aminopeptidase ou pour définir l'appartenance d'au moins une bactérie au groupe des Gram positifs ou des Gram négatifs selon sa coloration. L'invention concerne également des milieux de culture contenant de tels substrats, l'utilisation des substrats ou des milieux pour la détection d'activités aminopeptidases et/ou la différentiation de bactéries à Gram positif par rapport à des bactéries à Gram négatif et des procédés d'utilisation

A cet effet, la présente invention concerne des substrats chromogéniques enzymatiques pour la détection chez des micro-organismes d'une activité aminopeptidase ou pour définir l'appartenance d'au moins une bactérie au groupe des Gram positifs ou des Gram négatifs selon sa coloration. Ils ont la formule (I) suivante: 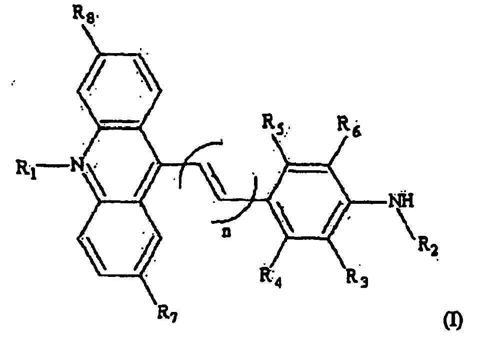 dans laquelle :
- R₁ est rien ou un groupement alkyle, allyle, aryle*,*
- R₂ est constitué par au moins un acide aminé, préférentiellement alanine,
- R₃, R₄, R₅ et R₆ sont constitués indépendamment l'un de l'autre par H ou -O-alkyle, préférentiellement -O-CH₃,
- R₇ est constitué par H, O-CH₃, alkyle ou halogène,
- R₈ est constitué par H ou Cl, et
- n est un chiffre entier correspondant à 0 ou 1.

Selon l'invention, on entend notamment par aryle un noyau aromatique en C₆-C₁₀, notamment phényle, benzyle, 1-naphtyle ou 2-naphtyle.

On entend par alkyle un alkyle en C₁-C₆, à savoir un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Par atome d'halogène, on entend le chlore, le brome, l'iode et le fluor.

Les acides aminés utilisés dans l'invention sont tout acide aminé connu de l'homme du métier.

Par au moins un acide aminé, on entend un ou plusieurs acides aminés.

Selon un mode de réalisation de l'invention, R₂ représente un acide aminé ou un peptide ayant au plus 10 acides aminés dans lequel les acides aminés sont identiques ou différents. De préférence, pour une question de coût du substrat, A représente un acide aminé ou un peptide ayant au plus 4 acides aminés dans lequel les acides aminés sont identiques ou différents, de préférence identiques.

Le ou les acides aminés R₂ peuvent être couplés à un agent de blocage, ce qui constitue un autre mode de réalisation de l'invention.

Ces agents de blocage comprennent tout agent de blocage connu de l'homme du métier qui est capable de protéger les aminés. A titre d'exemple, on peut citer le t-butoxycarbonyle (N-tBOC), le 9-fluorényloxycarbonyle, un agent de solubilisation tel que le succinyle, ou bien un acide aminé non métabolisable, c'est-à-dire non naturel, tel que l'acide pipécolique.

Selon un mode de réalisation, le substrat a la formule (Ia) suivante : 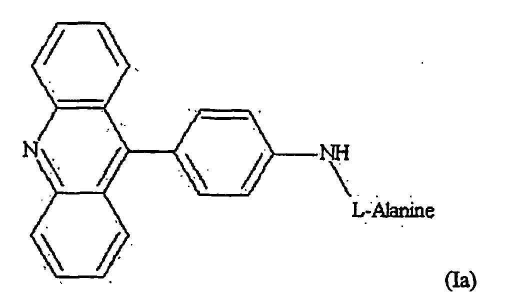 ou il a la formule (Ib) suivante :

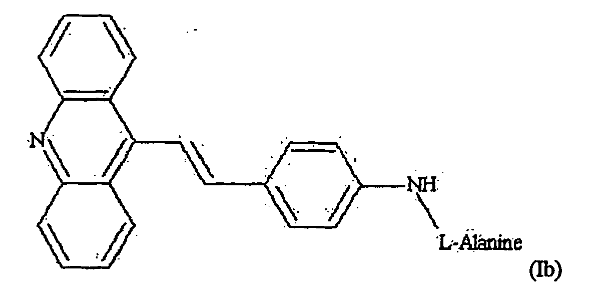

Selon un autre mode de réalisation, le substrat répond à la formule (I) ci-dessus dans laquelle R₁ est un groupement méthyle ou allyle,

Selon encore un autre mode de réalisation, le substrat répond à la formule (I) ci-dessus dans laquelle R₁ est un groupement alkyle, de préférence méthyle, ou un groupement allyle, R₂ est au moins un acide aminé, éventuellement bloqué avec un agent de blocage, de préférence au moins une alanine, R₃, R₄, R₅, R₆, R₇ et R₈ sont H, et n est 0 ou 1.

Selon encore un autre mode de réalisation, le substrat a la formule (Ic) suivante : 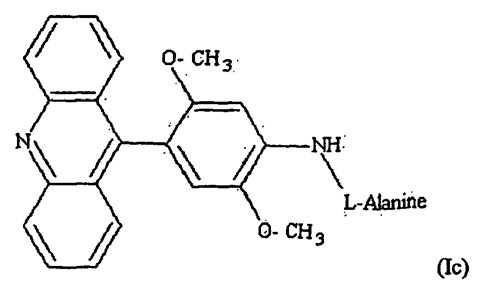 ou il a la formule (Id) suivante :

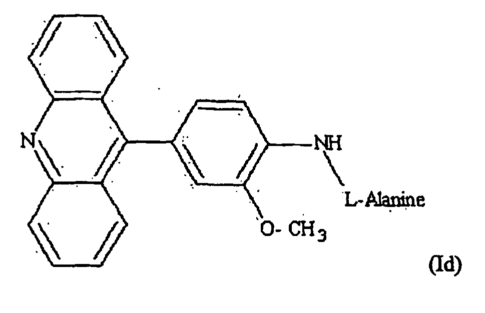

Les composés de l'invention peuvent être préparés en fonction de la valeur de n, comme suit :
(1) Quand n=0, on met à réagir avec de l'acridine chlorhydrique appropriée (R₈ est H ou Cl) avec de l'aniline appropriée (R₃, R₄, R₅ et R₆ étant tels que définis précédemment) et du soufre pour obtenir la 9-(4-aminophényl)acridine appropriée. Cette dernière est ensuite mise à réagir avec au moins un acide aminé couplé à un agent de blocage et le composé obtenu est éventuellement déprotégé pour séparer l'agent de blocage du composé de l'invention. Lorsqu'on veut obtenir un composé pour lequel l'azote en position 10 est quartemisé, on met à réagir le composé obtenu avec un XR₁ dans laquelle X est un halogène et R₁ est tel que défini précédemment, et
(2) Quand n=1, on met à réagir de la 9-méthylacridine, préparée selon la méthode de Campbell et al. (1958, *supra*), ou de la 9-chloroacridine, préparée selon la méthode de Lehmstedt et Schrader (Albert, 1966, *supra*), avec du nitrobenzaldéhyde approprié (R₃, R₄, R₅ et R₆ étant tels que définis précédemment) et du chlorure de zinc pour préparer de la 9-(4-nitrostyryl)acridine appropriée. Le groupement nitro est ensuite réduit soit par du chlorure d'étain (II) dans un mélange d'acétate d'éthyle et d'éthanol, soit par du borhydrate de sodium et de l'acétylacétonate de cuivre, pour obtenir la 9-(4-aminostyryl)acridine. Cette dernière est ensuite mise à réagir avec au moins un acide aminé couplé à un agent de blocage et le composé obtenu est éventuellement déprotégé pour séparer l'agent de blocage du composé de l'invention. Lorsqu'on veut obtenir un composé pour lequel l'azote en position 10 est quartemisé, on met à réagir le composé obtenu avec un XR₁ dans laquelle X est un halogène et R₁ est tel que défini précédemment.

L'invention concerne également un milieu de culture utilisant au moins un substrat chromogénique enzymatique, tel que décrit ci-dessus, seul ou en combinaison avec au moins un autre substrat enzymatique spécifique d'une activité enzymatique différente d'une activité de celle détectée par le substrat selon l'invention.

En effet, lorsque des microorganimes exprimant une activité peptidase sont ensemencés dans un milieu réactionnel contenant les composés de l'invention, il se produit une coloration ne diffusant pas dans le milieu réactionnel, et donc concentrée au niveau des colonies.

Par milieu réactionnel selon l'invention, on entend un milieu permettant le développement d'au moins une activité enzymatique d'au moins un microorganisme.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.

Le milieu réactionnel peut être solide, semi-solide ou liquide. Par milieu solide, on entend par exemple un milieu gélifié.

Préférentiellement, ce milieu est constitué par un milieu gélifié.

L'agar est l'agent gélifiant traditionnel en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

La quantité d'agar dans le milieu réactionnel est de 2 à 40g/l et de préférence de 9 à 25g/l.

Les substrats enzymatiques de l'invention sont utilisables dans une large gamme de pH, notamment entre pH 5,5 et 10.

La concentration de substrat enzymatique de l'invention dans le milieu réactionnel est comprise entre 0,025 et 1,0 g/l et, avantageusement, elle est de 0,3 g/l. En effet, à cette concentration de substrat, on obtient un meilleur contraste de coloration.

Le milieu réactionnel peut comprendre au moins un autre substrat spécifique d'une activité enzymatique différente de celle détectée par le substrat selon l'invention. L'hydrolyse enzymatique du ou des autres substrats génère un signal détectable, différent du signal détecté par le substrat de l'invention, comme par exemple des produits colorés ou fluorescents différents, pour permettre la mise en évidence comme la détection et/ou l'identification et/ou la quantification d'un ou plusieurs microorganismes.

A titre d'autre substrat spécifique, on peut utiliser tout autre substrat classiquement utilisé dans la détection des microorganismes.

La concentration de l'autre substrat enzymatique spécifique est généralement comprise entre 0,01 et 2 g/l. L'homme du métier pourra déterminer facilement une telle concentration en fonction du substrat utilisé.

Le milieu réactionnel peut également comprendre un ou plusieurs éléments en combinaison, tels que des acides aminés, des peptones, des hydrates de carbone, des nucléotides, des minéraux, des vitamines, des antibiotiques, des tensioactifs, des tampons, des sels de phosphate, d'ammonium, de sodium, de métaux. Des exemples de milieux sont décrits dans les demandes de brevet de la Demanderesse, EP 656 421 et WO99/09 207.

Les substrats enzymatiques et milieux réactionnels de l'invention sont donc utiles dans le diagnostic de microorganismes à activité peptidase.

Ainsi, la présente invention a trait également à l'utilisation des substrats chromogéniques enzymatiques, tels que décrits ci-dessus, ou d'un milieu de culture, également décrit ci-dessus, pour la détection chez des micro-organismes d'au moins une activité aminopeptidase.

La présente invention a toujours pour objet l'utilisation des substrats chromogéniques enzymatiques, tels que décrits ci-dessus, ou d'un milieu de culture, également décrit ci-dessus, pour séparer les bactéries à coloration à Gram positif des bactéries à coloration à Gram négatif

L'invention concerne enfin un procédé pour la détection chez des micro-organismes d'au moins une activité aminopeptidase, ce procédé consiste à :
- disposer d'un milieu de culture, tel que défini précédemment,
- ensemencer le milieu avec un échantillon biologique à tester,
- laisser incuber, et
- révéler la présence d'au moins une activité aminopeptidase seule ou en combinaison avec au moins une autre activité enzymatique différente.

L'invention concerne aussi un autre procédé pour la différentiation chez des bactéries de leur appartenance aux germes du type Gram positif ou aux germes du type Gram négatif, ce procédé consiste à :
- disposer d'un milieu de culture, tel que défini précédemment,
- ensemencer le milieu avec un échantillon biologique à tester,
- laisser incuber, et
- révéler la présence d'au moins une coloration synonyme de la présence de germe(s) du type Gram négatif

Quel que soit le procédé utilisé, lorsque l'azote en position 10 du groupement acridine n'est pas quaternisé, la révélation de la présence d'au moins une activité aminopeptidase est réalisée par l'ajout d'acide, préférentiellement d'acide chlorhydrique, d'acide acétique ou d'acide citrique, sur la culture. Par quaternisé, il faut comprendre que l'azote en position 10 du groupement acridine est tétravalent, c'est-à-dire qu'il est lié par trois liaisons classiques avec le cycle phényle et une liaison complémentaire avec un radical, de sorte que ledit atome d'azote est porteur d'une charge positive et est donc cationique. Dans ce cas, la molécule est sous la forme d'un sel, par exemple un sel de chlorure, bromure ou trifluoroacétate.

Toutes les réactions, qui vont être décrites ci-dessous dans les exemples, ont été suivies en chromatographie sur couche mince (CCM) et les structures des produits ont été confirmées par spectrométrie de masse (MS) et résonance magnétique nucléaire (RMN).

### Exemple 1 : Révélation de l'activité L-Alanine-aminopeptidase de micro-organismes sur milieu gélifié à l'aide de substrats non quaternisés :

### 1.1 : Molécules utilisées :

On a réalisé un étude comparative de deux substrats à base d'acridine la L-Alanyl-9-(4-aminostyryl)acridine, ci-après référencée L-Ala-4-ASA, et la L-Alanyl-aminophenylacridine (substrats non quatemisés), ci-après référencée L-Ala-APA, de formules respectives :

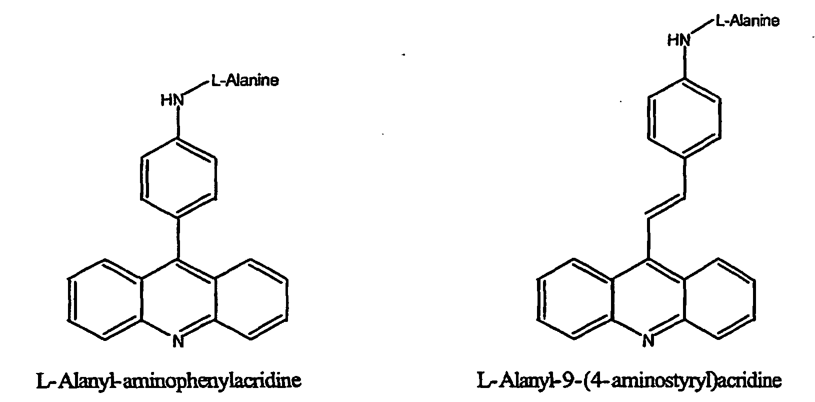

### 1.2 : Synthèse des substrats :

### 1.2.1 : Synthèse de la L-Ala-4-ASA :

Cette synthèse s'effectue en plusieurs étapes.

### Préparation de 9-chloroacridine :

La préparation est effectuée en utilisant la méthode de Lehmstedt et Schrader qui a été référencée par Albert dans son livre ' The Acridines ', Arnold, second edition, (1966), page 33.

### Préparation de 9-méthylacridine

La méthode de Campbell, Franklin, Morgan et Tivey (réf. J. Chem. Soc., 1958, 1145) a été utilisée. Les rendements ont atteint 90%.

### Préparation de 9-(4-nitrostyryl)acridine par fusion

Un mélange de 9-méthylacridine (4,83 g, 25,0 mmole), 4-nitrobenzaldéhyde (4,23 g, 31,25 mmole) et chlorure de zinc (5,06 g, 31,25 mmole) est chauffé à 130°C pendant 3 heures avec un bain d'huile. Le solide récupéré est chauffé dans une solution de sodium metabisulfite pour éliminer l'excès de l'aldéhyde et le mélange chaud est filtré. Des précipités obtenus sont dissous dans une quantité minimum de tétrahydrofurane et de l'eau est ajoutée dans la solution pour avoir le produit sous forme de précipités. Ces précipités sont récupérés par une filtration et séchés. Le produit peut être recristallisé dans l'éthanol. Le rendement est de 35%.

### Préparation de 9-(4-aminostyryl)acridine

Le produit 9-(4-nitrostyryl)acridine (2,86 g, 10,0 mmole) est dissous dans de l'acétate d'éthyle (250ml), puis porté au reflux. Une solution de chlorure d'étain (II) dihydraté (9,0g, 40mmmol) dans de l'éthanol chaud (150ml) est refroidit et ajouté à la solution de 9-(4-nitrostyryl) acridine. L'ensemble est mis à réagir sous reflux et sous agitation pendant 5 heures. Après refroidissement, la 9-(4-aminostyryl)acridine précipite et est isolée par filtration sous vide. Le filtrat est séché par évaporation et repris dans de l'eau (500ml) sous agitation. La solution est alcalinisée (pH 13-14) avec une solution de soude. La fraction de 9-(4-aminostyryl)acridine précédemment isolée qui contient une forte proportion de sel d'étain est également alcalinisée. La 9-(4-aminostyryl)acridine est séparée par filtration, lavée à l'eau et séchée. Elle est suffisamment pure pour l'étape suivante.

### Préparation de t-Boc-alanyl-9-(4-aminostyryl)acridine

Le produit t-Boc-alanine (3,79 g, 20,0 mmole) est dissous dans une quantité minimum de tétrahydrofurane (THF) anhydride et la solution est refroidie à -15°C à l'aide d'un mélange éthylène glycol/carbone glace dans un bain. La N-méthylmorpholine (NMM) (2,02 g, 20 mmole) est ajoutée goutte à goutte dans le mélange. Le chloroformiate d'isobutyle (IBCF) (2,53 g, 20,0 mmole) est ensuite introduit goutte à goutte dans le mélange réactionneL Il faut que la température soit au-dessous de -10°C. Au bout de 3 minutes environ, la 9-(4-aminostyryl)acridine (5,4 g, 20 mmole), qui est dissoute dans une quantité minimum de tétrahydrofurane (THF) anhydride et refroidie à -15°C, est introduite. Le mélange réactionnel est agité et on le laisse revenir à la température ambiante. Le sel de N-méthylmorpholine est éliminé par une filtration avec un entonnoir à plaque filtrante. Le mélange est évaporé jusqu'à son quatrième volume original avec un évaporateur rotatif. Le filtrat est introduit goutte à goutte dans un mélange eau/glace en quantité importante sous agitation. Des précipités jaunes, formés, sont récupérés par une filtration, lavés avec de l'eau et séchés. Le produit peut être recristallisé dans le méthanol. Le rendement est de sensiblement 75 %.

D'autres analogues des acides aminés protégés par le t-Boc ont aussi été utilisés pour former de nouveaux produits. Les acides aminés sont : la proline, la glycine, la sérine et la β-alanine. Les rendements pour ces produits sont dans la zone de 60 à 80 %, les résultats étant généralement les meilleurs pour les analogues de la β-alanine et les moins bons pour les analogues de la sérine.

### 1.2.2 : Synthèse de la L-Ala-APA :

La préparation de 9-(4-aminophényl)acridine par une réaction de soufre fondu peut être réalisée par deux méthodes différentes.

### Méthode A

L'acridine chlorhydrique (9,7 g, 45,0 mmole), l'aniline (8,37 g, 90,0 mmole) et 10,0 g de soufre sont bien mélangés et chauffés à 130°C pendant 4 heures sous une hotte efficace. Le milieu réactionnel dans le ballon est refroidi à température ambiante et dissous dans le méthanol chaud pour donner une solution de couleur rouge sanguine. La solution est refroidie et le soufre est éliminé par une filtration. L'alcanilisation du filtrat est réalisée à l'aide d'une solution d'ammoniaque concentrée. Des précipités jaunes légers, formés, sont filtrés puis lavés avec le méthanol froid. Le rendement est de 65%.

### Méthode B

L'acridine (8,06 g, 45,0 mmole), l'aniline chlorhydrique (11,61 g, 90 mmole) et 10,0 g de soufre sont bien mélangés et chauffés à 130°C pendant 4 heures. Le milieu réactionnel est ensuite traité comme dans la Méthode A ci-dessus.
Ensuite on réalise une préparation sur la base de l'une et/ou l'autre des deux méthodes précédentes.

### Préparation de t-Boc-alanyl-9-(4-aminophényl) acridine

Le produit t-Boc-alanine (3,79 g, 20,0 mmole) est dissous dans une quantité minimum de tétrahydrofurane (THF) anhydride et la solution est refroidie à -15°C à l'aide d'un mélange éthylène glycol/carbone glace dans un bain. La N-méthylmorpholine (NMM) (2,02 g, 20 mmole) est ajoutée goutte à goutte dans le mélange. Le chloroformiate d'isobutyle (IBCF) (2,53 g, 20,0 mmole) est ensuite introduit goutte à goutte dans le mélange réactionnel. Il faut que la température soit au-dessous de -10°C. Au bout de 3 minutes environ, la 9-(4-aminophényl)acridine (5,4 g, 20 mmole), qui est dissoute dans une quantité minimum de tétrahydrofurane (THF) anhydride et refroidie à -15°C, est introduite. Le mélange réactionnel est agité et on le laisse revenir à la température ambiante. Le sel de N-méthylmorpholine est éliminé par une filtration avec un entonnoir à plaque filtrante. Le mélange est évaporé jusqu'à son quatrième volume original avec un évaporateur rotatif. Le filtrat est introduit goutte à goutte dans un mélange eau/glace en quantité importante sous agitation. Des précipités jaunes, formés, sont récupérés par une filtration, lavés avec de l'eau et séchés. Le produit peut être recristallisé dans le méthanol. Le rendement est de 75%.

D'autres analogues des acides aminés protégés par le t-Boc ont aussi été utilisés pour former de nouveaux produits. Les acides aminés sont : la proline, la glycine, la sérine et la β-alanine. Les rendements pour ces produits sont dans la zone de 60 à 80% : les résultats étant généralement les meilleurs pour les analogues de la β-alanine et les moins bons pour les analogues de la sérine.

### 1.3 : Préparation du milieu :

Un milieu gélifié comprenant de la L-Alanyl-9-(4-aminostyryl)acridine (ci-après référencée L-Ala-4-ASA) ou de la L-Alanyl-aminophenylacridine (ci-après référencée L-Ala-APA) est préparé comme suit : 46,37 g d'agar Columbia sont additionnés à 1 litre d'eau distillée puis autoclavés. Ce milieu réactionnel est séparé en deux milieux de volume équivalent. Chacun de ces milieux comprend respectivement: 0,3 g/l de L-Ala-4-ASA apporté par une solution mère dans du DMSO et 0,3 g/l de L-Ala-APA apporté aussi par une solution mère dans du DMSO.

Des micro-organismes issus de la collection de la Demanderesse ont été ensemencés sur chacun des milieux par isolement en trois cadrans à partir d'une suspension à 0,5 McFarland. Les boîtes ont été incubées à 37 °C pendant 24 heures. Les colonies formées ont été examinées visuellement après 24 heures d'incubation. La coloration de ces colonies a été notée avant et après ajout d'une goutte d'acide chlorhydrique.

### 1.4 : Résultats :

Les résultats sont présentés dans le tableau 1 ci-dessous. En absence d'HCl, les deux substrats ne donnent pas de coloration spontanée. En présence d'une goutte d'HCl, les colonies possédant l'activité L-Alanine-aminopeptidase sont colorées en gris-mauve à mauve. Les colonies ne possédant pas cette activité demeurent incolores. Ces substrats sont donc sensibles et spécifiques. Ils peuvent permettre dans le cas d'une culture mixte Gram positif et Gram négatif de séparer les deux types de germes.

**Tableau 1 : Révélation de l'activité L-Alanine-aminopeptidase de micro-organismes par la L-Ala-4-ASA ou la L-Ala-APA sur milieu gélifié**

| ESPECES (N° souche interne) | 0,3 g/l | 0,3 g/l | 0,3 g/l | 0,3 g/l |
|---|---|---|---|---|
| | L-Ala-4-ASA | L-Ala-APA | L-Ala-4-ASA | L-Ala-APA |
| | sans HCl | sans HCl | avec HCl | avec HCl |
| *Escherichia coli* | incolore | incolore | gris mauve | mauve pâle |
| *Proteus mirabilis* | incolore | incolore | gris mauve | mauve très pâle pâle |
| *Klebsiella pneumoniae* | incolore | incolore | gris mauve | mauve |
| *Enterobacter cloacae* | incolore | incolore | gris mauve | mauve |
| *Citrobacter koseri* | incolore | incolore | gris mauve | mauve |
| *Streptococcus agalactiae* | incolore | incolore | incolore | incolore |
| *Enterococcus faecalis* | incolore | incolore | incolore | incolore |
| *Staphylococcus aureus* | incolore | incolore | incolore | incolore |
| *Listeria innocua* | incolore | incolore | incolore | incolore |
| *Candida albicans* | incolore | incolore | incolore | incolore |

### Exemple 2 : Révélation de l'activité L-Alanine-aminopeptidase de micro-organismes sur milieu gélifié à l'aide de substrat quaternisé :

### 2.1 : Molécule utilisée :

L'activité L-Alanine-aminopeptidase de micro-organismes sur milieu gélifié est réalisée par l'utilisation du chlorure de L-Alanyl-9(4-aminophenyl)-10-allyl-acridinium (substrat quaternisé), ci-après désigné L-Ala-4-AP-10-AA, de formule :

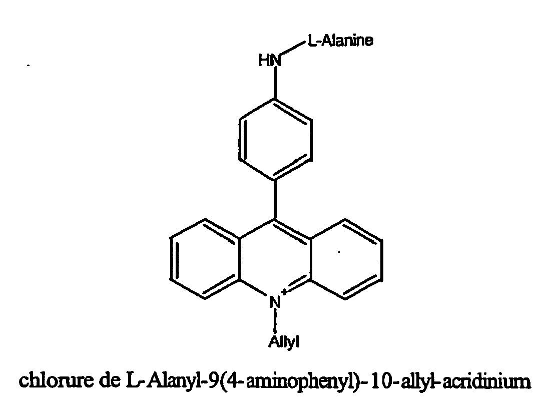

### 2.2 : Synthèse de L-Ala-4-AP-10-AA :

Le composé de départ est la t-Boc-alanyl-9-(4-aminophényl)acridine dont la synthèse a déjà été exposée au paragraphe 1.22 (Synthèse de L-Ala-APA). Dans un flacon de 10 ml qui peut être fermé, la t-Boc-alanyl-9-(4-aminophényl)acridine (0,88 g, 2,0 mmole) est introduite dans le tétrahydrofurane anhydride (4,0 ml) et le mélange est mis en l'ébullition pour avoir une solution partielle. La solution / suspension est refroidie et le bromure d'allyle (2,0 ml) est ajouté. Le flacon est ensuite porté au reflux pendant 8 heures, et la réaction est régulièrement suivie par chromatographie sur couche mince (CCM). Après refroidissement et élimination du solvant, le sel quaternaire est lavé avec de l'éther diéthylique et séché.

Le produit est dissous dans une quantité minimum d'éthanol et agité avec l'acétate d'éthyle (10 ml) saturé par HCL Des précipités, qui sont formés quelques heures après, sont récupérés par une filtration sous pression réduite. L'introduction de l'éther éthylique dans le filtrat permet de récupérer le produit en plus. Les fractions réunies du produit sont lavées avec l'éther éthylique et séchées rapidement pour éviter l'absorption d'humidité.

### 2.3 : Préparation du milieu :

Un milieu gélifié comprenant comme substrat du L-Ala-4-AP-10-AA est préparé comme suit : 46,37 g d'agar Columbia sont additionnés à 1 litre d'eau distillée puis autoclavés. Ce milieu réactionnel est séparé en trois milieux de volume équivalent. Chacun de ces milieux comprend respectivement: 0,1 g/l, 0,2 g/l, 0,4 g/l de L-Ala-4-AP-10-AA apporté par une solution mère dans du DMSO.

Des micro-organismes issus de la collection de la Demanderesse ont été ensemencés sur chacun des milieux par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées.

### 2.4 : Résultats :

Les résultats sont exprimés en intensité de coloration en se basant sur une échelle arbitraire allant de 0 à 4. Ces résultats sont présentés dans le tableau 2 ci-dessous.

Ce substrat permet de révéler une activité L-Alanine-aminopeptidase chez les bactéries à Gram négatif par l'apparition spontanée (sans ajout d'acide) d'une couleur concentrée au niveau des colonies. Les colonies ne possédant pas cette activité demeurent incolores. Ce substrat est donc sensible et spécifique. Il peut permettre dans le cas d'une culture mixte Gram positif et Gram négatif de séparer les deux types de germes. Le fait de « quatemiser » l'azote en position 10 du groupement acridine permet d'obtenir une réaction spontanée sans ajout d'acide comparativement à la même molécule non quaternisée décrite dans l'exemple 1.

**Tableau 2 : Influence de la concentration en substrat dans la révélation de l'activité L-Alanine-aminopeptidase de micro-organismes par désigné la L-Ala-4-AP-10-AA**

| SOUCHES | Temps d'incubation | 0,1 g/l de | | 0,2 g/l de | | 0,4 g/l de | |
|---|---|---|---|---|---|---|---|
| | | L-Ala-4- AP-10-AA | | L-Ala-4-AP-10-AA | | L-Ala-4-AP-10-AA | |
| | | Couleur | Intensité | Couleur | Intensité | Couleur | Intensité |
| *Escherichia coli* | 24 H | beige | 0,5 | rose-orange | 2 | rose-orange | 2,5 |
| (032) | 48 h | beige | 0,5 | rose-orange | 2 | rose-orange | 3 |
| *Proteus mirabilis* | 24 H | beige | traces | rose-orange | 0,5 | rose-orange | 3 |
| (103) | 48 h | beige | traces | rose-orange | 1 | rose-orange | 3 |
| *Klebsiella* | 24 H | beige | traces | rose-orange | 1 | rose-orange | 3 |
| *pneumoniae* (023) | 48 h | beige | traces | rose-orange | 2 | rose-orange | 3,5 |
| *Citrobacter koseri* | 24 H | beige | 0,5 | rose-orange | 1 | rose-orange | 3 |
| (090) | 48 h | beige | 0,5 | rose-orange | 2 | rose-orange | 3,5 |
| *Staphylococcus* | 24 H | inhibé | - | inhibé | - | inhibé | - |
| *aureus* (070) | 48 h | inhibé | - | inhibé | - | inhibé | - |
| *Streptococcus* | 24 H | inhibé | - | inhibé | - | inhibé | - |
| *pyogenes* (067) | 48 h | incolore | - | incolore | - | incolore | - |
| *Enterococcus* | 24 H | incolore | - | orange | traces | orange | traces |
| *faecalis* (075) | 48 h | incolore | - | orange | 0,5 | orange | 1 |
| *Listeria innocua* | 24 H | incolore | - | incolore | - | incolore | - |
| (036) | 48 h | incolore | - | incolore | - | incolore | - |
| *Candida albicans* | 24 H | incolore | - | incolore | - | incolore | - |
| (056) | 48 h | incolore | - | incolore | - | incolore | - |

### Exemple 3 : Révélation de l'activité L-Alanine-aminopeptidase de micro-organismes sur milieu gélifié à l'aide d'un autre substrat quaternisé :

### 3.1 : Molécule utilisée :

La révélation de l'activité L-Alanine-aminopeptidase de micro-organismes sur milieu gélifié s'effectue par l'utilisation d'un substrat appelé chlorure de L-Alanyl-9(4-aminophenyl)-10-methyl-acridinium (substrat quaternisé), ci-après appelé L-Ala-4-AP-10-MA, dont la formule est la suivante :

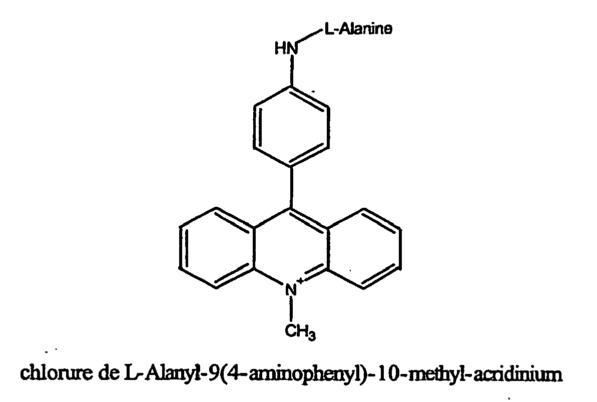

### 3.2 : Synthèse de L-Ala-4-AP-10-MA :

Cette synthèse a déjà été réalisée au paragraphe 2.2 ci-dessus, dans laquelle le bromure d'allyle a été remplacé par l'iodure de méthyle. Il convient de s'y reporter pour connaître la synthèse de la L-Ala-4-AP-10-MA.

### 3.3 : Préparation du milieu :

Un milieu gélifié comprenant 300 mg/l de L-Ala-4-AP-10-MA, est préparé comme suit : 46,37 g d'agar Columbia sont additionnés à 1 litre d'eau distillée puis autoclavés, le substrat est ensuite apporté par une solution mère dans du DMSO. Des micro-organismes issus de la collection de la Demanderesse ont été ensemencés sur le milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées.

### 3.4 : Résultats :

Les résultats sont présentés dans le tableau 3 ci-dessous :

**Tableau 3: Révélation de l'activité L-Alanine-aminopeptidase de micro-organismes par de la L-Ala-4-AP-10-AA dont la concentration en substrat est optimisée**

| SOUCHES | Temps d'incubation | 0,3 g/l de L-Ala-4-AP-10-MA | |
|---|---|---|---|
| | | Couleur | Intensité |
| *Escherichia coli* | 24 H | rose-orange | 3,5 |
| (032) | 48 h | rose-orange | 3,5 |
| *Proteus mirabilis* | 24 H | rose-orange | 1 |
| (103) | 48 h | rose-orange | 2 |
| *Klebsiella* | 24 H | rose-orange | 3 |
| *pneumoniae* (023) | 48 h | rose-orange | 3 |
| *Citrobacter koseri* | 24 H | rose-orange | 3 |
| (090) | 48 h | rose-orange | 3 |
| *Staphylococcus* | 24 H | inhibé | - |
| *aureus* (070) | 48 h | inhibé | - |
| *Streptococcus* | 24 H | inhibé | - |
| *pyogenes* (067) | 48 h | inhibé | - |
| *Enterococcus* | 24 H | rose | traces |
| *faecalis* (075) | 48 h | rose-orange | 0,5 |
| *Listeria innocua* | 24 H | incolore | - |
| (036) | 48 h | rose-orange | traces |
| *Candida albicans* | 24 H | incolore | - |
| (056) | 48 h | incolore | - |

Ce substrat permet de révéler une activité L-Alanine-aminopeptidase chez les bactéries à Gram négatif par l'apparition spontanée (sans ajout d'acide) d'une couleur rose-orange concentrée au niveau des colonies. Les colonies ne possédant pas cette activité demeurent incolores. Ce substrat est donc sensible et spécifique. Il peut permettre dans le cas d'une culture mixte Gram +/Gram - de séparer les deux types de germes. Comparativement au substrat décrit dans l'exemple 2, il semble permettre d'obtenir des intensités de coloration plus fortes pour les souches positives. La différence entre ces deux substrats est le type de groupement en position 10 sur l'azote du noyau d'acridine.

### Exemple 4 : Révélation de l'activité L-Alanine-aminopeptidase de micro-organismes sur milieu gélifié à l'aide d'autres substrats non quaternisés :

### 4.1 : Molécules utilisées :

La révélation de l'activité L-Alanine-aminopeptidase de micro-organismes sur milieu gélifié s'effectue par l'utilisation de deux substrats appelés L-Alanyl-2-methoxyaminophenylacridine, ci-après appelée L-Ala-2-MeOAPA, et L-Alanyl-2,5-dimethoxyaminophenylacridine, ci-après appelée L-Ala-2,5-diMeOAPA, dont les formules sont respectivement les suivantes :

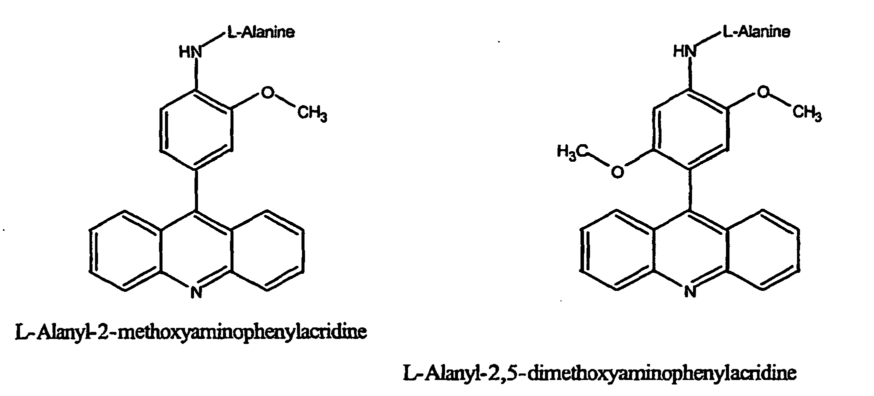

### 4.2 : Synthèse des substrats :

### 4.2.1 : Synthèse de L-Ala-2-MeOAPA :

Sur la base de ce qui est décrit au point 1.2.2 ci-dessus, d'autres analogues d'aniline sont aussi utilisés pour former des nouveaux produits, y compris l'anisidine (3-méthoxyaniline) qui donne la 9-(4-amino-2-méthoxyphényl)acridine.

### 4.2.2 : Synthèse de L-Ala-2,5-diMeOAPA :

Sur la base de ce qui est décrit au point 1.2.2 ci-dessus, d'autres analogues d'aniline sont aussi utilisés pour former des nouveaux produits, y compris la 2,5-diméthoxyaniline qui donne la 9-(4-amino-2,5-diméthoxyphényl)acridine.

### 4.3 : Préparation du milieu :

Un milieu gélifié comprenant de la L-Ala-2-MeOAPA ou de la L-Ala-2,5-diMeOAPA est préparé comme suit : 46,37 g d'agar Columbia sont additionnés à 1 litre d'eau distillée puis autoclavés. Ce milieu réactionnel est séparé en deux milieux de volume équivalent Chacun de ces milieux comprend respectivement : 0,3 g/l de L-Ala-2-MeOAPA apporté par une solution mère dans du DMSO et 0,3 g/l de L-Ala-2,5-diMeOAPA apporté aussi par une solution mère dans du DMSO. Des micro-organismes issus de la collection de la Demanderesse ont été ensemencés sur chacun des milieux par isolement en trois cadrans à partir d'une suspension à 0,5 McFarland. Les boîtes ont été incubées à 37 °C pendant 24 heures. La coloration de ces colonies ainsi que l'intensité de cette coloration ont été notées après ajout d'HCl.

### 4.4: Résultat :

Les résultats sont présentés dans le tableau 4 ci-dessous :

**Tableau 4: Révélation de l'activité L-Alanine-aminopeptidase de micro-organismes par les L-Ala-2-MeOAPA et L-Ala-2,5-diMeOAPA**

| SOUCHES | Temps d'incubation | L-Ala-2- MeOAPA | | L-Ala-2,5-di MeEOAPA | |
|---|---|---|---|---|---|
| | | Couleur | Intensité | Couleur | Intensité |
| *Escherichia coli* | 24 H | orange | 0,5 | jaune orange | 1,5 |
| (032) | 48 h | rose | 3,5 | jaune orange | 2 |
| *Proteus mirabilis* | 24 H | orange | 1 | incolore | 0, |
| (037) | 48 h | orange | 1,5 | orange | 0,5 |
| *Klebsiella* | 24 H | rose | 3 | orange | 1 |
| *pneumoniae* (023) | 48 h | rose | 3,5 | jaune orange | 2,5 |
| *Citrobacter koseri* | 24 H | orange | 2 | orange | 1 |
| (012) | 48 h | rose | 3,5 | jaune orange | 2,5 |
| *Enterobacter* | 24 H | rose | 2 | jaune orange | 1,5 |
| *cloacae* (061) | 48 h | rose | 2 | jaune orange | 2 |
| *Staphylococcus* | 24 H | incolore* | 0 | incolore | 0 |
| *aureus* (035) | 48 h | incolore* | 0 | incolore | 0 |
| *Streptococcus* | 24 H | inhibé | - | incolore | 0 |
| *agalactiae* (001) | 48 h | inhibé | - | incolore | 0 |
| *Enterococcus* | 24 H | inhibé | - | jaune orange | 0,5 |
| *faecalis* (117) | 48 h | inhibé | - | jaune orange | 3 |
| *Listeria innocua* | 24 H | incolore* | 0 | incolore | 0 |
| (036) | 48 h | incolore* | 0 | incolore | 0 |
| *Candida albicans* | 24 H | inhibé | - | incolore | 0 |
| (077) | 48 h | inhibé | - | incolore | 0 |

| | | | | | |
|---|---|---|---|---|---|
| * croissance très faible | | | | | |

Ces deux substrats permettent après ajout d'une goutte d'HCl de révéler une activité L-Alanine-aminopeptidase chez les bactéries à Gram positif. L'ajout de substituants (Méthoxy-) sur le groupement phényl permet en plus de réduire la toxicité des substrats, notamment vis-à-vis des bactéries à Gram positif. Cependant, ce gain en fertilité se fait au détriment de la spécificité, en effet, *Enterococcus faecalis* présente une activité avec la L-Ala-2,5-diMeOAPA après 48 heures d'incubation.

### Exemple 5 : Révélation de l'activité β-Alanine-aminopeptidase de micro-organismes sur milieu gélifié: utilisation du chlorure de β-Alanyll-9(4-aminophenyl))-10-methyl-acridinium (substrat quaternisé) :

### 5.1 : Molécule utilisée :

La révélation de l'activité β-Alanine-aminopeptidase de micro-organismes sur milieu gélifié s'effectue par l'utilisation d'un substrat appelé chlorure de β-Alanyl-9(4-aminophenyl)-10-methyl-acridinium (substrat quaternisé), ci-après appelé β-Ala-4-AP-10-MA, dont la formule est la suivante :

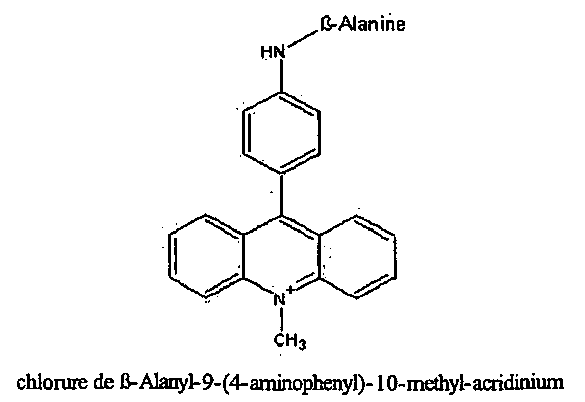

### 5.2 : Synthèse de β-Ala-4-AP-10-MA :

Le composé de départ est la t-Boc-alanyl-9-(4-aminophényl)acridine dont la synthèse a déjà été exposée au paragraphe 1.2.2 (Synthèse du L-Ala-APA). La t-Boc-alanyl-9-(4-aminophényl)acridine (0,67 g, 1,5 mmole) est dissoute dans l'acétonitrile (volume minimum) et chauffée au reflux avec de l'iodure de méthyle (4,0 ml) Le flacon est ensuite fermé et incubé à 40°C pendant plus de 100 heures. Le réactif solide est dissous au fur et à mesure en formant une solution orange. Des cristaux orangés - noirs apparaissent dans cette solution.

A l'issue des 100 heures, le mélange réactionnel est transféré dans l'acétate d'éthyle (100 ml) sous agitation. Après une durée nécessaire, le sel quaternaire est filtré et lavé avec l'éther diéthylique.

Le produit est dissous dans une quantité minimum d'éthanol et agité avec l'acétate d'éthyle (10 ml) saturé par HCl. Des précipités, qui sont formés quelques heures après, sont récupérés par une filtration sous pression réduite. L'introduction de l'éther diéthylique dans le filtrat permet de récupérer le produit en plus. Les fractions réunies du produit sont lavées avec l'éther diéthylique et séchées rapidement pour éviter l'absorption d'humidité.

La déprotection est la même que celle décrite précédemment

### 5.3 : Préparation du milieu :

Un milieu gélifié comprenant 300 mg/l de β-Ala-4-AP-10-MA est préparé comme suit : 46,37 g d'agar Cblumbia sont additionnés à 1 litre d'eau distillée puis autoclavés, le substrat est ensuite apporté par une solution mère dans du DMSO. Des micro-organismes issus de la collection de la Demanderesse ont été ensemencés sur le milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées.

### 5.4 : Résultats :

Les résultats sont présentés dans le tableau 5 ci-dessous.

Ce substrat permet de révéler une activité β-Alanine-aminopeptidase chez les bactéries à Gram négatif par l'apparition spontanée (sans ajout d'acide) d'une couleur orange concentrée au niveau des colonies. Les colonies ne possédant pas cette activité demeurent incolore. Ce substrat est donc sensible et spécifique. Il peut permettre d'identifier les souches de *Pseudomonas aeruginosa* et notamment de les différencier des autres bactéries.

**Tableau 5 : Révélation de l'activité β-Alanine-aminopeptidase de micro-organismes par de la β-Ala-4-AP-10-MA**

| SOUCHES | Temps d'incubation | 0,3 g/l de β-Ala-4-AP-10-MA | |
|---|---|---|---|
| | | Couleur | Intensité |
| *Pseudomonas* | 24 H | orange | 2 |
| *aeruginosa* (052) | 48 h | orange | 3 |
| *Pseudomonas* | 24 H | orange | 1,5 |
| *aeruginosa* (165) | 48 h | orange | 3 |
| *Burkholderia* | 24 H | incolore | - |
| *cepacia* (004) | 48 h | incolore | - |
| *Pseudomonas* | 24 H | inhibé | - |
| *fluorescens* (016) | 48 h | incolore | - |
| *Pseudomonas* | 24 H | inhibé | - |
| *stutzeri* (073) | 48 h | incolore | - |
| *Pseudomonas* | 24 H | incolore | - |
| *putida* (028) | 48 H | incolore | - |
| *Acinetobacter* | 24 H | incolore | - |
| *calcoaceticus (034)* | 48 H | incolore | - |
| *Escherichia coli* | 24 H | incolore | - |
| (032) | 48 H | incolore | - |
| *Klebsiella* | 24 H | incolore | - |
| *pneumoniae* (023) | 48 H | incolore | - |

### Exemple 6 : Révélation de l'activité L-Proline-aminopeptidase de micro-organismes sur milieu gélifié à l'aide d'un substrat non quaternisé à base de Proline :

### 6.1 : Molécule utilisée :

La révélation de l'activité L-Proline-aminopeptidase de micro-organismes sur milieu gélifié s'effectue par l'utilisation d'un substrat appelé L-Prolyl-9(4-aminophenyl)-acridine (substrat non quaternisé), ci-après appelée L-Pro-4-APA, dont la formule est la suivante :

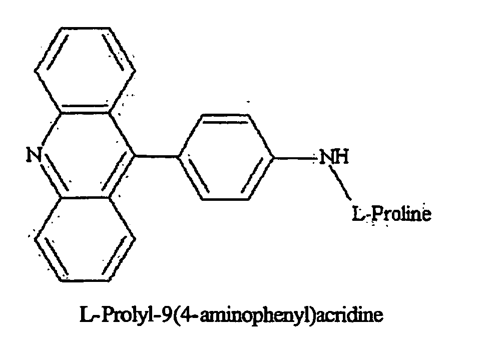

### 6.2 : Synthèse de L-Pro-4-APA :

Cette synthèse a déjà été réalisée au paragraphe 1.2 ci-dessus, selon deux méthodes A et B, dans lesquelles l'acide aminé Alanine a été remplacé par l'acide aminé Proline. Il convient de s'y reporter pour connaître la synthèse de la L-Pro-4-APA.

### 6.3 : Préparation du milieu :

Un milieu gélifié comprenant de la L-Prolyl-9-(4-aminophenyl)acridine (ci-après référencée L-Pro-4-APA) est préparé comme suit : 45g d'agar Sabouraud sont additionnés à 1 litre d'eau distillée puis autoclavés. Ce milieu réactionnel comprend 0,3 g/l de L-Pro-APA apporté aussi par une solution mère dans du DMSO.

Des micro-organismes issus de la collection de la Demanderesse ont été ensemencés sur ce milieu par isolement en trois cadrans à partir d'une suspension à 0,5 McFarland. Les boîtes ont été incubées à 37 °C pendant 24 heures. Les colonies formées ont été examinées visuellement après 24 heures d'incubation. La coloration de ces colonies a été notée avant et après ajout d'une goutte d'acide acétique, ci-après GAA (glacial acetic acid).

### 6.4 : Résultats :

Les résultats sont présentés dans le tableau 6 ci-dessous :

**Tableau 6 : Révélation de l'activité L-Proline-aminopeptidase de micro-organismes par de la L-Pro-4-APA dont la concentration en substrat est optimisée**

| SOUCHES | Goutte d'Acide Acétique | 0,3 g/l de L-Pro-4-APA | |
|---|---|---|---|
| | | Couleur | Intensité |
| *Candida albicans* | avant GAA | jaune | 2 |
| (138) | après GAA | jaune | 3 |
| *Candida* | avant GAA | blanc | - |
| *parapsilosis* (040) | après GAA | jaune | 2 |
| *Candida* | avant GAA | pas de croissance | - |
| *lusitaniae (045)* | après GAA | pas de croissance | - |
| *Candida* | avant GAA | pas de croissance | - |
| *guilliermondii (046)* | après GAA | pas de croissance | - |
| *Candida krusei* | avant GAA | jaune | 1 |
| (026) | après GAA | jaune | 3 |
| *Candida glabrata* | avant GAA | jaune | 2 |
| (051) | après GAA | jaune | 3 |

Avec certaines souches de levures *Candida,* la nature inhibitrice du substrat est démontrée. Cependant, certaines levures produisent une coloration jaune, sans l'addition d'acide. Cette coloration est plus intense pour certaines souches, notamment *Candida albicans,* elle est également plus intense qu'en l'absence de culture. Ceci démontre bien que notre substrat, contenant la Proline comme acide aminé, permet de révéler l'activité L-Proline-aminopeptidase chez les levures.

### Exemple 7 : Révélation de l'activité L-Sérine-aminopeptidase de micro-organismes sur milieu gélifié à l'aide d'un substrat non quaternisé à base de Sérine :

### 7.1 : Molécule utilisée :

La révélation de l'activité L-Sérine-aminopeptidase de micro-organismes sur milieu gélifié s'effectue par l'utilisation d'un substrat appelé L-Séryl-9(4-aminophenyl)-acridine (substrat non quaternisé), ci-après appelée L-Ser-4-APA, dont la formule est la suivante :

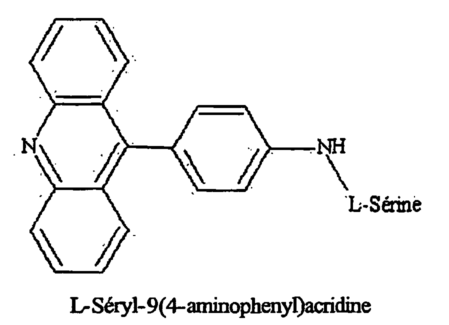

### 7.2 : Synthèse de L-Ser-4-APA :

A l'instar de ce qui a été exposé pour la LPro-4-APA (paragraphe 6.2 ci-dessus), cette synthèse a déjà été réalisée au paragraphe 1.2 ci-dessus, selon deux méthodes A et B, dans lesquelles l'acide aminé Alanine a été remplacé par l'acide aminé Sérine. Il convient de s'y reporter pour connaître la synthèse de la L-Ser-4-APA.

### 7.3 : Préparation du milieu :

Un milieu gélifié comprenant de la L-Seryl-9(4-aminophenyl)acridine (ci-après référencé L-Ser-4-APA) est préparé comme suit : 30 milligrammes de L-Ser-4-APA sont ajoutés à 4 grammes d'agar Columbia, additionnés à 0,1 litre d'eau distillée puis autoclaves. Ce milieu réactionnel est mis à l'autoclave à 116°C pendant 20 minutes. La majeure partie du substrat se retrouve en solution, sans coloration résiduelle.

Des micro-organismes issus de la collection de la Demanderesse ont été ensemencés sur ce milieu à partir d'une suspension à 0,5 McFarland. Les boîtes ont été incubées à 37 °C pendant 24 heures. Des échantillons de 10 µl de chaque suspension sont ensuite cultivés pour produire des colonies. Les colonies formées ont été examinées visuellement après 18 à 24 heures d'incubation.

La coloration de ces colonies a été notée avant et après ajout d'une goutte d'acide acétique, ci-après GAA (glacial acetic acid).

### 7.4 : Résultats :

Les résultats sont présentés dans le tableau 7 ci-dessous :

**Tableau 7 : Révélation de l'activité L-Sérine-aminopeptidase de micro-organismes par de la L-Ser-4-APA dont la concentration en substrat est optimisée**

| SOUCHES | GAA | 0,3 g/l de L-Ser-4-APA | |
|---|---|---|---|
| | | Couleur | Intensité |
| *Escherichia coli* | avant GAA | crème | - |
| (009) | après GAA | orange pâle | 2 |
| *Klebsiella* | avant GAA | crème | - |
| *pneumoniae* (012) | après GAA | orange pâle | 2 |
| *Yersinia* | avant GAA | crème | - |
| *enterocolitica* (061) | après GAA | orange pâle | 2 |
| *Candida albicans* | avant GAA | crème | - |
| (138) | après GAA | crème | - |
| *Staphylococcus* | avant GAA | crème | - |
| *aureus* (008) | après GAA | crème | - |
| *Enterococcus* | avant GAA | crème | - |
| *faecalis* (117) | après GAA | crème | - |

Encore une fois, certaines souches ont une inhibition partielle avec le substrat à base d'acridine. Les trois souches à Gram négatif testées produisent une coloration réactionnelle particulière après l'addition d'acide acétique.

### Exemple 8 : Révélation de l'activité L-alanine-aminopeptidase de micro-organismes sur milieu gélifié à l'aide de substrats quaternisés à base de une, deux ou trois L-alanine :

### 8.1 : Molécule utilisée :

La révélation de l'activité L-alanine-aminopeptidase de micro-organismes sur milieu gélifié s'effectue par l'utilisation de trois substrats, à savoir la L-Ala-4-AP- 10-MA telle que décrite dans l'exemple 3, la L-Ala-L-Ala-4-AP-10-MA qui correspond à la L-Ala-4-AP-10-MA comprenant une L-alanine supplémentaire, et la L-Ala-L-Ala-L-Ala-4-AP-10-MA qui possède encore une L-Alanine supplémentaire.

### 8.2 : Synthèse de ces substrats :

Ces substrats ont été préparés comme décrits dans le point 3.2 ci-dessus à partir du composé de départ approprié possédant une, deux ou trois L-Alanines.

### 8.3 : Préparation du milieu :

Un milieu gélifié comprenant ces trois substrats est préparé comme suit : 30 mg de chaque substrat sont dissous (chauffage) dans 10 ml d'eau distillée stérile. Dix ml de cette solution sont ensuite ajoutés à 90 ml d'agar Columbia maintenus en surfusion à 50°C.

Diverses souches de microorganismes issues de la collection NCRC (National Collection of Type Cultures, Colindales, UK) ont été ensemencées sur les milieux ainsi obtenus selon l'inoculation multi-point : pour chaque souche, une goutte de 10µl d'une suspension à 0,5 McFarland est déposée sur chacun des milieux de culture.

Les colonies formées ont été examinées visuellement après 24h et 48h d'incubation. La coloration et la croissance de ces colonies ont été notées.

### 8.4 : Résultats :

Les résultats sont présentés dans le tableau 8 ci-dessous :

| SOUCHES (Référence) | L-Ala-4-AP-10-MA | | L-Ala-L-Ala-4-AP-10-MA | | L-Ala-L-Ala-L-Ala-4-AP-10-MA | |
|---|---|---|---|---|---|---|
| | 24h | 48h | 24h | 48h | 24h | 48h |
| *E. coli 0157* | Orange | Orange | Orange | Orange | Orange | Orange |
| (NCTC 12079) | (++) | (++) | (++) | (++) | (++) | (++) |
| *Salmonella typhimurium* | Orange | Orange | Orange | Orange | Orange | Orange |
| (NCTC 74) | (++) | (++) | (++) | (++) | (++) | (++) |
| *Salomonella Poona* | Orange | Orange | Orange | Orange | Orange | Orange |
| (NCTC 4840) | (++) | (++) | (++) | (++) | (++) | (++) |
| *Shigella sonnei* | Orange | Orange | Orange | Orange | Orange | Orange |
| (NCTC 9774) | (++) | (++) | (++) | (++) | (++) | (++) |
| *Pseudomonas aeruginosa* | Orange | Orange | Orange | Orange | Orange | Orange |
| (NCTC 10662) | (++) | (++) | (++) | (++) | (++) | (++) |
| *Klebsiella pneumoniae* | Orange | Orange | Orange | Orange | Orange | Orange |
| (NCTC 10896) | (++) | (++) | (++) | (++) | (++) | (++) |
| *Enterobacter cloacae* | Orange | Orange | Orange | Orange | Orange | Orange |
| (NCTC 11936) | (++) | (++) | (++) | (++) | (++) | (++) |
| *Burkholderia cepacia* | Orange | Orange | Orange | Orange | Orange | Orange |
| (NCTC 10743) | (++) | (++) | (++) | (++) | (++) | (++) |
| *Serratia marcescens* | Orange | Orange | Orange | Orange | Orange | Orange |
| (NCTC 10211) | (++) | (++) | (++) | (++) | (++) | (++) |
| *Enterococcus faecalis* | PC | PC | Incolore | Incolore | Incolore | Incolore |
| (NCTC 755) | | | (++) | (++) | (++) | (++) |
| *Enterococcus faecalis* | Incolore | Incolore | Incolore | Incolore | Incolore | Incolore |
| (NCTC 12697) | (+/-) | (+/-) | (++) | (++) | (++) | (++) |
| *Enterococcus faecium* | Incolore | Incolore | Incolore | Incolore | Incolore | Incolore |
| (NCTC 7171) | (++) | (++) | (++) | (++) | (++) | (++) |
| *Listeria monocytogenes* | Incolore | Incolore | Incolore | Incolore | Incolore | Incolore |
| (NCTC 11994) | (++) | (++) | (++) | (++) | (++) | (++) |
| *Staphylococcus aureus* | PC | PC | PC | PC | Incolore | Incolore |
| (NCTC 6571) | | | | | (++) | (++) |
| *Staphylococcus aureus* | Incolore | Incolore | Incolore | Incolore | Incolore | Incolore |
| (NCTC 11939) | (++) | (++) | (++) | (++) | (++) | (++) |

| | | | | | | |
|---|---|---|---|---|---|---|
| ++ signifie bonne croissance, + signifie croissance modérée, +/- signifie faible croissance et PC signifie pas de croissance | | | | | | |

Sur les trois milieux, les souches de bactéries à Gram négatif ont formé des colonies colorées en orange, alors que les souches de bactéries à Gram positif ont formé des colonies incolores ou ne se sont pas développées. Ces trois milieux permettent donc de différencier les bactéries à Gram négatif de celles à Gram positif, et suivant les milieux, la croissance de tout ou partie des souches testées.

### Exemple 9 : Révélation de l'activité Pyroglutamine-aminoneptidase de micro-organismes sur milieu gélifié à l'aide de substrats quaternisés à base de sel de chorhydrate de pyroglutamyl-9-(4-aminophényl)-10-méthyl-acridine :

### 9.1 : Molécule utilisée :

La révélation de l'activité pyroglutamine-aminopeptidase de micro-organismes sur milieu gélifié s'effectue par l'utilisation de pyroglutamyl-9-(4-aminophényl)-10-méthylacridine, ci-après appelée PG-4-AP-10-MA.

### 9.2 : Synthèse de ce substrat :

Ce substrat a été préparé comme décrit dans le point 3.2 ci-dessus à ceci près qu'on a remplacé la L-alanine par la pyroglutamine.

### 9.3 : Préparation du milieu :

Un milieu gélifié comprenant ce substrat est préparé comme suit : 30 mg de chaque substrat sont dissous (chauffage) dans 10 ml d'eau distillée stérile. Dix ml de cette solution sont ensuite ajoutés à 90 ml d'agar Columbia maintenus en surfusion à 50°C.

Diverses souches de microorganismes issues de la collection NCTC (National Collection of Type Cultures, Colindales, UK) ont été ensemencées sur les milieux ainsi obtenus selon l'inoculation multi-point décrite dans l'exemple 8 ci-dessus.

Les colonies formées ont été examinées visuellement après 24h et 48h d'incubation. La coloration et la croissance de ces colonies ont été notées.

### 9.4 : Résultats :

Les résultats sont présentés dans le tableau 9 ci-dessous :

| SOUCHE (référence) | PG-4-AP-10-MA | |
|---|---|---|
| | 24h | 48h |
| *Escherichia coli (NCTC* 14018) | Incolore (++) | Incolore (++) |
| *Escherichia coli 0157* (NCTC 12079) | Incolore (++) | Incolore (++) |
| *Salomonella typhimurium* (NCTC 74) | Incolore (++) | Incolore (++) |
| *Salmonella poona* (NCTC 4840) | Incolore (++) | Incolore (++) |
| *Shigella sonnei* (NCTC 9774) | Incolore (++) | Incolore (++) |
| *Pseudomonas aeruginosa* (NCTC 10662) | Incolore (++) | Orange (++) |
| *Klebsiella pneumonia* (NCTC 10896) | Incolore (++) | Orange pale (++) |
| *Enterobacter cloacae* (NCTC 11936) | Incolore (++) | Orange pale (++) |
| *Burkholderia cepacia* (NCTC 10743) | Incolore (++) | Orange pale (++) |
| *Serratia marcescens* (NCTC 10211) | Incolore (++) | Orange pale (++) |

| | | |
|---|---|---|
| ++ signifie bonne croissance, + signifie croissance modérée, +/- signifie faible croissance et PC signifie pas de croissance | | |

Le milieu de l'exemple permet de différencier les bactéries exprimant une activité pyroglutamyl-aminopeptidase de celles ne l'exprimant pas.

### Exemple 10 : Révélation de l'activité glycine-aminopeptidase de micro-organismes sur milieu gélifié à l'aide d'un substrat quaternisé à base de sel de chlorhydrate de glycyl-9-(4-aminophényl)-10-méthyl-acridine :

### 10.1 : Molécule utilisée :

La révélation de l'activité glycine-aminopeptidase de micro-organismes sur milieu gélifié s'effectue par l'utilisation de glycyl-9-(4-aminophényl)-10-médlyl-acridine, ci-après appelée G-4-AP-10-MA.

### 10.2 : Synthèse de ce substrat :

Ce substrat a été préparé comme décrit dans le point 3.2 ci-dessus à ceci près qu'on a remplacé la L-alanine par la glycine.

### 10.3 : Préparation du milieu :

Un milieu gélifié comprenant ce substrat est préparé comme suit : 30 mg du substrat sont dissous (chauffage) dans 10 ml d'eau distillée stérile. Dix ml de cette solution sont ensuite ajoutés à 90 ml d'agar Columbia maintenus en surfusion à 50°C.

Diverses souches de microorganismes issues de la collection NCTC (National Collection of Type Cultures, Colindales, UK) ont été ensemencées sur les milieux ainsi obtenus selon l'inoculation multi-point décrite dans l'exemple 8 ci-dessus.

Les colonies formées ont été examinées visuellement après 24h et 48h d'incubation. La coloration et la croissance de ces colonies ont été notées.

### 10.4 : Résultats :

Les résultats sont présentés dans le tableau 10 ci-dessous :

| SOUCHE (référence) | G-4-AP-10-MA | |
|---|---|---|
| | 24h | 48h |
| *Escherichia coli* (NCTC 14018) | PC | PC |
| *Escherichia coli O157* (NCTC 12079) | Orange (++) | Orange (++) |
| *Salomonella typhimurium* (NCTC 74) | Orange pale (++) | Orange pale (++) |
| *Salmonella poona* (NCTC 4840) | Orange (++) | Orange (++) |
| *Shigella sonnei* (NCTC 9774) | Orange (++) | Orange (++) |
| *Pseudomonas aeruginosa* (NCTC 10662) | Orange pale (++) | Orange (++) |
| *Klebsiella pneumonia* (NCTC 10896) | Orange (++) | Orange (++) |
| *Enterobacter cloacae* (NCTC 11936) | Orange (++) | Orange (++) |
| *Brirkholderia cepacia* (NCTC 10743) | Orange (++) | Orange (++) |
| *Serratia marcescens* (NCTC 10211) | Orange (++) | Orange (++) |
| *Enterococcus faecalis* (NCTC 755) | PC | PC |
| *Enterococcus faecalis* (NCTC 12697) | Incolore (+) | Incolore (+) |
| *Enterococcus faecium* (NCTC 7171) | Incolore (+) | Incolore (+) |
| *Listeria monocytogenes* (NCTC 11994) | Incolore (+) | Incolore (+) |
| *Staphylococcus aureus* (NCTC 6571) | PC | PC |
| *Staphylococcus aureus* (NCTC 11939) | Incolore (+) | Incolore (+) |

| | | |
|---|---|---|
| ++ signifie bonne croissance, + signifie croissance modérée, +/- signifie faible croissance et PC signifie pas de croissance | | |

Le milieu de l'exemple permet de différencier les bactéries exprimant une activité glycylamyl-aminopeptidase de celles ne l'exprimant pas.

### Exemple 11 : Révélation de l'activité t-BOC-L-alanyl-L-alanyl-L-alanyl-pentidase de micro-organismes sur milieu gélifié à l'aide d'un substrat quaternisé pour lequel l'acide aminé est couplé à un agent de blocage, substrat à base de sel de chlorhydrate de t-BOC-L-alanyl-L-alanyl-L-alanyl-9-(4-aminophényl)-10-méthyl-acridine:

### 11.1 : Molécule utilisée :

La révélation de l'activité L-alanine-aminopeptidase de micro-organismes sur milieu gélifié s'effectue par l'utilisation de t-BOC-L-alanyl-L-alanyl-L-alanyl-9-(4-aminophényl)-10-méthyl-acridine, ci-après appelée t-BOC-L-Ala-4-AP-10-MA

### 11.2 : Synthèse de ce substrat :

Ce substrat a été préparé comme décrit dans le point 3.2 ci-dessus à ceci près qu'on n'a pas procédé à la déprotection du composé aminé.

### 11.3 : Préparation du milieu :

Un milieu gélifié comprenant ce substrat est préparé comme suit : 30 mg du substrat sont dissous (chauffage) dans 10 ml d'eau distillée stérile. Dix ml de cette solution sont ensuite ajoutés à 90 ml d'agar Columbia maintenus en surfusion à 50°C.

Diverses souches de microorganismes issues de la collection NCTC (National Collection of Type Cultures, Colindales, UK) ont été ensemencées sur les milieux ainsi obtenus selon l'inoculation multi-point décrite dans l'exemple 8 ci-dessus.

Les colonies formées ont été examinées visuellement après 24h et 48h d'incubation. La coloration et la croissance de ces colonies ont été notées.

### 11.4 : Résultats :

Les résultats sont présentés dans le tableau 11 ci-dessous :

| SOUCHE (référence) | t-BOC-L-Ala-Ala-Ala-4-AP-10-MA | |
|---|---|---|
| | 24h | 48h |
| *Escherichia coli 0157* (NCTC 12079) | Orange pale (++) | Orange pale (++) |
| *Salomonella typhimurium* (NCTC 74) | Orange pale (++) | Orange pale (++) |
| *Salnzonella poona* (NCTC 4840) | Orange pale (++) | Orange pale (++) |
| *Shigella sonnei* (NCTC 9774) | Orange pale (++) | Orange pale (++) |
| *Pseudomonas aeruginosa* (NCTC 10662) | Orange pale (++) | Orange pale (++) |
| *Klebsiella pneumonia* (NCTC 10896) | Orange (++) | Orange (++) |
| *Enterobacter cloacae* (NCTC 11936) | Orange (++) | Orange (++) |
| *Burkholderia cepacia* (NCTC 10743) | Orange (++) | Orange (++) |
| *Serratia marcescens* (NCTC 10211) | Orange (++) | Orange (++) |
| *Enterococcus faecalis* (NCTC 755) | PC | PC |
| *Enterococcus faecalis* (NCTC 12697) | Incolore (+/-) | Incolore (+/-) |
| *Enterococcus faecium* (NCTC 7171) | Incolore (+/-) | Incolore (++) |
| *Listeria monocytogenes* (NCTC 11994) | Incolore (+/-) | Incolore (+) |
| *Staphylococcus aureus* (NCTC 6571) | PC | PC |
| *Staphylococcus aureus* (NCTC 11939) | Incolore (+) | Incolore (+) |

| | | |
|---|---|---|
| ++ signifie bonne croissance, + signifie croissance modérée, +/- signifie faible croissance et PC signifie pas de croissance | | |

Le milieu de l'exemple permet d'obtenir quatre groupes de microorganismes :
- ceux formant des colonies colorées en orange franc,
- ceux formant des colonies colorées en orange pâle,
- ceux formant des colonies incolores et
- ceux ne formant pas de colonie.

Parmi les souches testées, les bactéries à Gram négatif appartiennent aux deux premiers groupes, alors que les bactéries à Gram positif appartiennent aux deux derniers.

La présence d'un agent de blocage (t-Boc) permet de moduler l'activité par rapport aux résultats obtenus avec le substrat déprotégé (voir exemple 8 ci-dessus).

### Autres expériences réalisées :

D'autres substrats ont été testés qui permettent de valider les résultats présentés ici, on peut par exemple citer :
- chlorure de L-Alanyl-9-(4-amino-3-methoxyphenyl)-10-methylacridinium,
- chlorhydrate de chlorure de L-Alanyl-9-(4-aminophenyl)-10-methylacridinium
- chlorhydrate de chlorure de L-Alanyl 9-(4-amino-2,5-dimethoxyphenyl)-10-methylacridinium,
- chlorhydrate de chlorure de β-Alanyl 9-(4-aminophenyl)-10-methylacridinium,
- β-Alanyl-9-(4-amino-2-methoxy-aminophenyl) acridine,
- β-Alanyl-9-(4-amino-2,5-dimethoxy-aminophenyl) acridine,
- chlorhydrate de chlorure de β-Alanyl 9-(4-amino-3-medioxyphenyl)-10-methylacridimium
- chlorhydrate de chlorure de β-Alanyl 9-(4-amino-2,5-dimethoxyphenyl)-10-methylacridinium.

De même d'autres espèces de micro-organismes, généralement des bactéries, ont également été testés, telles que :
- *Salmonella typhimurium,*
- *Staphylococcus epidermidis,*
- *Serratia marcescens.*

## Revendications

1. Substrats chromogéniques enzymatiques pour la détection chez des micro-organismes d'une activité aminopeptidase ou pour définir l'appartenance d'au moins une bactérie au groupe des Gram positifs ou des Gram négatifs selon sa coloration, **caractérisés par le fait qu'**ils ont la formule (I) suivante : 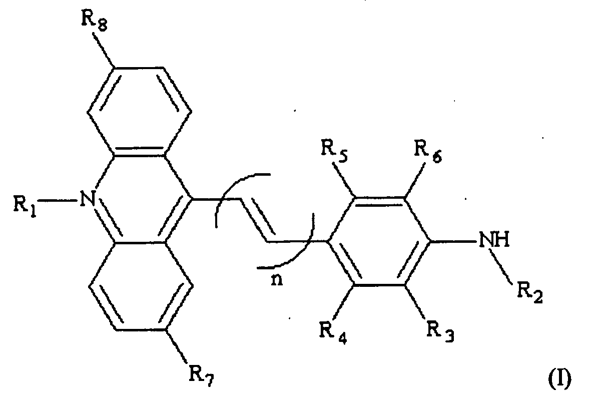 dans laquelle :
• R₁ est rien ou un groupement alkyle, allyle, aryle,
• R₂ est constitué par au moins un acide aminé, préférentiellement alanine,
• R₃, R₄, R₅ et R₆ sont constitués indépendamment l'un de l'autre par H- ou -O-alkyle, préférentiellement -O-CH₃,
• R₇ est constitué par H, O-CH₃, alkyle ou halogène,
• R₈ est constitué par H ou Cl, et
• n est un chiffre entier correspondant à 0 ou 1.

2. Substrats, selon la revendication 1, **caractérisé par le fait qu'**ils ont la formule suivante (Ia) : 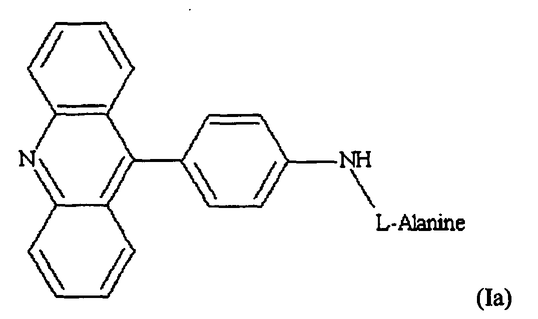 ou qu'ils ont la formule (Ib) suivante :

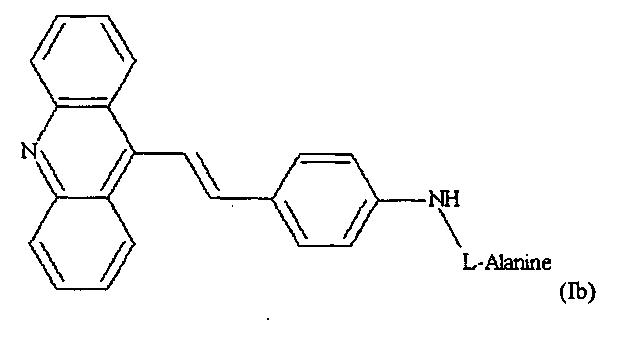

3. Substrats, selon la revendication 1, **caractérisés par le fait que** R₁ est un groupement méthyle ou allyle.

4. Substrats, selon la revendication 1, **caractérisés par le fait qu'**il a la formule (Ic) suivante : 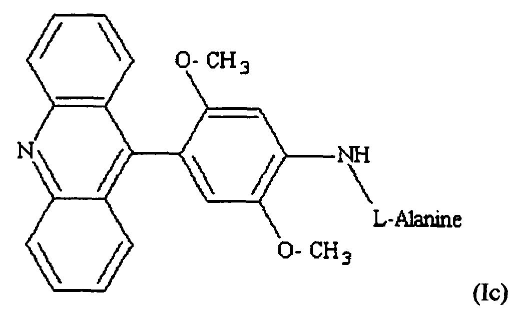 ou qu'il a la formule (Id) suivante :

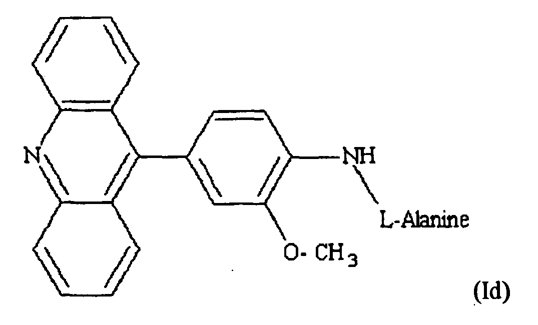

5. Substrats selon l'une quelconque des revendications 1 à 4, **caractérisé par le fait que** R₂ ou la L-alanine est couplé à un agent de blocage.

6. Milieu de culture comprenant au moins un substrat chromogénique enzymatique, selon l'une quelconque des revendications 1 à 5, seul ou en combinaison avec au moins un autre substrat enzymatique spécifique d'une activité enzymatique différente de celle détectée par le substrat selon l'invention.

7. Milieu, selon la revendication 6, **caractérisé par le fait qu'**il est constitué par un milieu gélifié.

8. Utilisation des substrats chromogéniques enzymatiques, tels que définis dans l'une quelconque des revendications 1 à 5, ou d'un milieu de culture, selon l'une quelconque des revendications 6 ou 7, pour la détection chez des micro-organismes d'au moins une activité aminopeptidase.

9. Utilisation des substrats chromogéniques enzymatiques, tels que définis dans l'une quelconque des revendications 1 à 5, ou d'un milieu de culture, selon l'une quelconque des revendications 6 ou 7, pour séparer les bactéries à coloration à Gram positif des bactéries à coloration à Gram négatif.

10. Procédé pour la détection chez des micro-organismes d'au moins une activité aminopeptidase, **caractérisé en ce qu'**il consiste à :
• disposer d'un milieu de culture, selon l'une quelconque des revendications 6 ou 7,
• ensemencer le milieu avec un échantillon biologique à tester,
• laisser incuber, et
• révéler la présence d'au moins une activité aminopeptidase seule ou en combinaison avec au moins une autre activité enzymatique différente d'une activité aminopeptidase.

11. Procédé pour la différentiation chez des bactéries de leur appartenance aux germes du type Gram positif ou aux germes du type Gram négatif, **caractérisé en ce qu'**il consiste à :
• disposer d'un milieu de culture, selon l'une quelconque des revendications 6 ou 7,
• ensemencer le milieu avec un échantillon biologique à tester,
• laisser incuber, et
• révéler la présence d'au moins une coloration synonyme de la présence de germe(s) du type Gram négatif.

12. Procédé, selon l'une quelconque des revendications 10 ou 11, **caractérisé en ce que**, lorsque l'azote en position 10 du groupement acridine n'est pas quaternisé, la révélation de la présence d'au moins une activité aminopeptidase est réalisée par l'ajout d'acide, préférentiellement d'acide chlorhydrique, d'acide acétique ou d'acide citrique, sur la culture.

## Claims

1. Chromogenic enzyme substrates for detecting aminopeptidase activity in microorganisms or for determining whether at least one bacterium belongs to the Gram-positive group or to the Gram-negative group according to the color thereof, **characterized in that** they have formula (I) below: 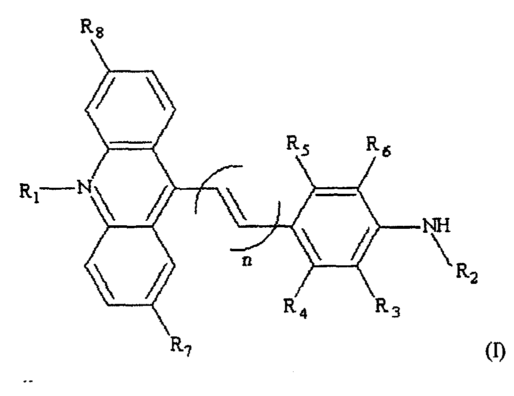 in which:
• R₁ is nothing or an alkyl, allyl or aryl group,
• R₂ consists of at least one amino acid, preferably alanine,
• R₃, R₄, R₅ and R₆ consist, independently of one another, of H- or -O-alkyl, preferably -O-CH₃,
• R₇ consists of H, O-CH₃, alkyl or halogen,
• R₈ consists of H or Cl, and
• n is an integer corresponding to 0 or 1.

2. The substrates as claimed in claim 1, **characterized in that** they have formula (Ia) below: 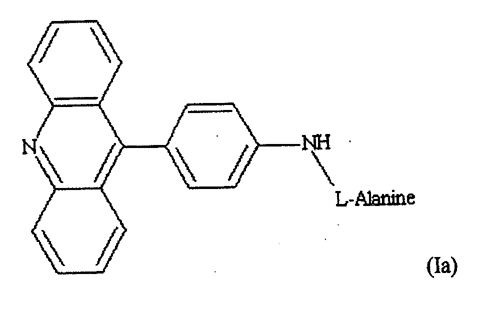 or **in that** they have formula (Ib) below:

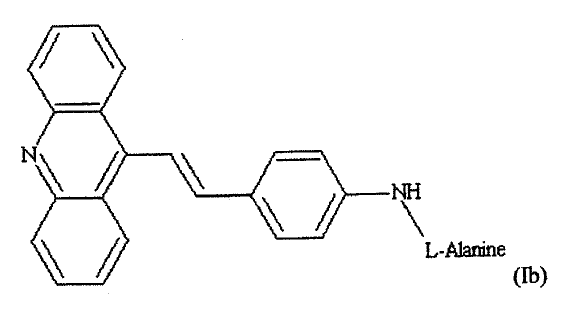

3. The substrates as claimed in claim 1, **characterized in that** R₁ is a methyl or allyl group.

4. The substrates as claimed in claim 1, **characterized in that** they have formula (Ic) below: 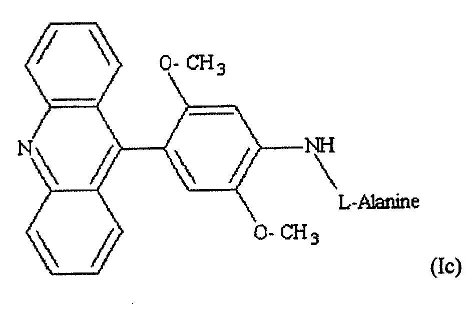 or **in that** they have formula (Id) below:

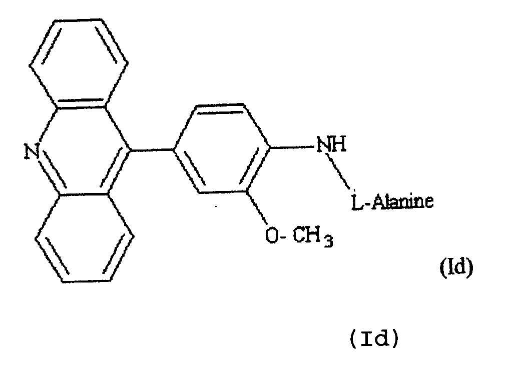

5. The substrates as claimed in any one of claims 1 to 4, **characterized in that** R₂ or the L-alanine is coupled to a blocking agent.

6. A culture medium comprising at least one chromogenic enzyme substrate as claimed in any one of claims 1 to 5, alone or in combination with at least one other enzyme substrate specific for an enzyme activity that is other than that detected by the substrate according to the invention.

7. The medium as claimed in claim 6, **characterized in that** it consists of a gelled medium.

8. The use of the chromogenic enzyme substrates as defined in any one of claims 1 to 5, or of a culture medium as claimed in either one of claims 6 and 7, for detecting at least one aminopeptidase activity in microorganisms.

9. The use of the chromogenic enzyme substrates as defined in any one of claims 1 to 5, or of a culture medium as claimed in either one of claims 6 and 7, for separating bacteria with Gram-positive coloration from bacteria with Gram-negative coloration.

10. A method for detecting at least one aminopeptidase activity in microorganisms, **characterized in that** it consists in:
• providing a culture medium as claimed in either one of claims 6 and 7,
• seeding the medium with a biological sample to be tested,
• leaving it to incubate, and
• visualizing the presence of at least one aminopeptidase activity, alone or in combination with at least one other enzyme activity different from an aminopeptidase activity.

11. A method for differentiating bacteria in terms of whether they belong to microorganisms of the Gram-positive type or to microorganisms of the Gram-negative type, **characterized in that** it consists in:
• providing a culture medium as claimed in either one of claims 6 and 7,
• seeding the medium with a biological sample to be tested,
• leaving it to incubate, and
• visualizing the presence of at least one color synonymous with the presence of a microorganism or microorganisms of the Gram-negative type.

12. The method as claimed in either one of claims 10 and 11, **characterized in that**, when the nitrogen in the 10-position of the acridine group is not quaternized, the presence of at least one aminopeptidase activity is visualized by adding acid, preferably hydrochloric acid, acetic acid or citric acid, to the culture.

## Patentansprüche

1. Chromogene Enzymsubstrate zum Nachweis einer Aminopeptidaseaktivität bei Mikroorganismen und zum Definieren der Zugehörigkeit mindestens einer Bakterie zur Gruppe der Grampositiven oder der Gramnegativen entsprechend ihrer Färbung, **dadurch gekennzeichnet, dass** sie die folgende Formel (I) haben: 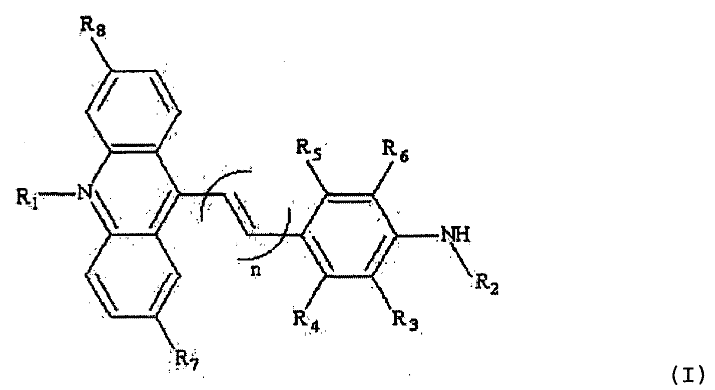 in der:
. R₁ nichts oder eine Alkyl-, Allyl-, Arylgruppe ist,
. R₂ aus mindestens einer Aminosäure, vorzugsweise Alanin, aufgebaut ist,
. R₃, R₄, R₅ und R₆ unabhängig voneinander aus H- oder -O-Alkyl, vorzugsweise -O-CH₃, aufgebaut sind,
. R₇ aus H, O-CH₃, Alkyl oder Halogen aufgebaut ist,
. R₈ aus H oder Cl aufgebaut ist, und
. n eine ganze Zahl ist, die 0 oder 1 entspricht.

2. Substrate nach Anspruch 1, **dadurch gekennzeichnet, dass** sie die folgende Formel (Ia) haben: 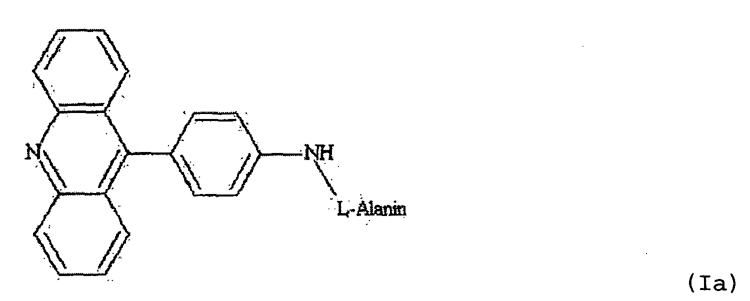 oder dass sie die folgende Formel (Ib) haben:

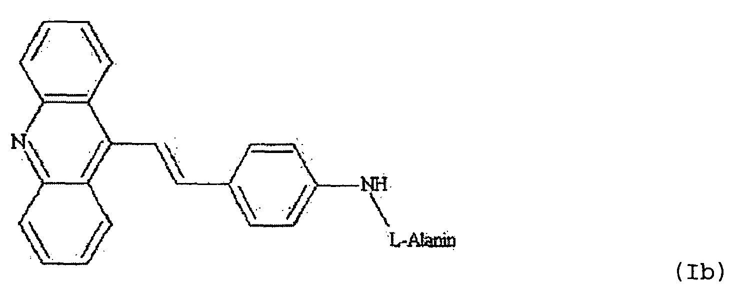

3. Substrate nach Anspruch 1, **dadurch gekennzeichnet, dass** R₁ eine Methyl- oder Allylgruppe ist.

4. Substrate nach Anspruch 1, **dadurch gekennzeichnet, dass** sie die folgende Formel (Ic) haben: 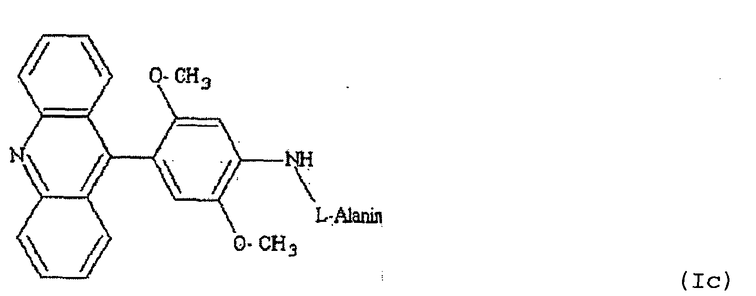 oder dass sie die folgende Formel (Id) haben:

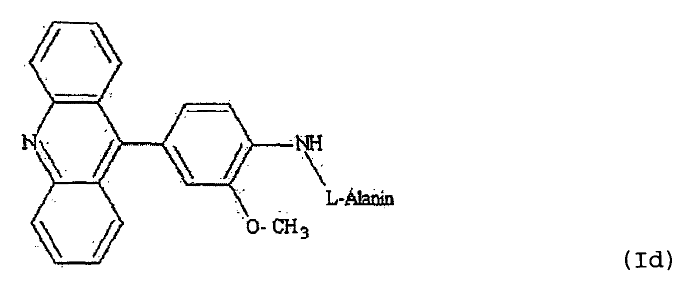

5. Substrate nach einem der Ansprüche 1 bis 4, **dadurch gekennzeichnet, dass** R₂ oder das L-Alanin an einen Blocker gekuppelt ist.

6. Kulturmedium, mit mindestens ein chromogenes Enzymsubstrat nach einem der Ansprüche 1 bis 5, allein oder in Kombination mit mindestens einem weiteren Enzymsubstrat, das für eine Enzymaktivität spezifisch ist, die von jener, die durch das erfindungsgemäße Substrat nachgewiesen wird, verschieden ist.

7. Medium nach Anspruch 6, **dadurch gekennzeichnet, dass** es aus einem Gelmedium gebildet ist.

8. Verwendung chromogener Enzymsubstrate wie in einem der Ansprüche 1 bis 5 definiert oder eines Kulturmediums nach einem der Ansprüche 6 oder 7 zum Nachweis mindestens einer Aminopeptidaseaktivität bei Mikroorganismen.

9. Verwendung chromogener Enzymsubstrate wie in einem der Ansprüche 1 bis 5 definiert oder eines Kulturmediums nach einem der Ansprüche 6 oder 7, um die Bakterien mit positiver Gramfärbung von den Bakterien mit negativer Gramfärbung zu unterscheiden.

10. Verfahren zum Nachweis mindestens einer Aminopeptidaseaktivität bei Mikroorganismen, **dadurch gekennzeichnet, dass** es umfasst:
. Bereitstellen eines Kulturmediums nach einem der Ansprüche 6 oder 7,
. Beimpfen des Mediums mit einer zu testenden biologischen Probe,
. Inkubierenlassen, und
. Sichtbarmachen des Vorhandenseins mindestens einer Aminopeptidaseaktivität, allein oder in Kombination mit mindestens einer weiteren Enzymaktivität, die von einer Aminopeptidaseaktivität verschieden ist.

11. Verfahren zum Differenzieren von Bakterien nach ihrer Zugehörigkeit zu Keimen vom grampositiven Typ oder zu Keimen vom gramnegativen Typ, **dadurch gekennzeichnet, dass** es umfasst:
. Bereitstellen eines Kulturmediums nach einem der Ansprüche 6 oder 7,
. Beimpfen des Mediums mit einer zu testenden biologischen Probe,
. Inkubierenlassen, und
. Sichtbarmachen des Vorhandenseins mindestens einer Färbung, die gleichbedeutend mit dem Vorhandensein von einem Keim bzw. von Keimen vom gramnegativen Typ ist.

12. Verfahren nach einem der Ansprüche 10 oder 11, **dadurch gekennzeichnet, dass** dann, wenn der Stickstoff an der 10er-Position der Acridingruppe nicht quaternisiert ist, das Sichtbarmachen des Vorhandenseins mindestens einer Aminopeptidaseaktivität durch Zugabe von Säure, vorzugsweise Salzsäure, Essigsäure oder Citronensäure, zu der Kultur erreicht wird.
